(19) Europäisches Patentamt
European Patent Office
Office européen des brevets

(11) Publication number: **0 326 637 B1**

(12) # EUROPEAN PATENT SPECIFICATION

(45) Date of publication of patent specification: **23.09.92**

(51) Int. Cl.5: **C07D 317/40**, A61K 31/335

(21) Application number: **88101607.5**

(22) Date of filing: **04.02.88**

(54) **New 2-anilinophenylacetic acid derivative.**

(43) Date of publication of application:
**09.08.89 Bulletin 89/32**

(45) Publication of the grant of the patent:
**23.09.92 Bulletin 92/39**

(84) Designated Contracting States:
**CH DE ES FR GB IT LI NL**

(56) References cited:
**EP-A- 0 070 013**

(73) Proprietor: **Mikasa Seiyaku Co., Ltd.**
**2-3, Toyotama-kita Nerima-ku**
**Tokyo(JP)**

Proprietor: **Daito Kabushiki Kaisha**
**326, Youkamachi**
**Toyama-shi, Toyama(JP)**

(72) Inventor: **Shimizu, Keisuke**
**160-455, Takano Ichishicho**
**Ichishi-gun Mie-ken(JP)**
Inventor: **Matsumura, Takumi**
**441, Kusajima**
**Toyama-shi Toyama-ken(JP)**
Inventor: **Nakamoto, Masato**
**No. 205, Dainiomachikopo 77-1, Nanbu Ikku**
**Omachi Toyama-shi Toyama-ken(JP)**

(74) Representative: **Vossius & Partner**
**Siebertstrasse 4 P.O. Box 86 07 67**
**W-8000 München 86(DE)**

EP 0 326 637 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to a new 2-anilinophenylacetic acid derivative possessing long-lasting and potent valuable pharmacological effects with less side-effects and to a process for preparing same. More particularly, the present invention relates to (5-methyl-2-oxo-1,3-dioxolen-4-yl)methyl 2-(2,6-dichloroanilino)-phenylacetate possessing long lasting and potent valuable pharmacological properties accompanied with an extremely low level of side-effects and to a process for preparing same.

In the past, a great number of non-steroidal drugs possessing anti-inflammatory, anitpyretic, analgesic and/or anti-rheumatic activity have been developed. The drugs developed hitherto are generally classified into several groups; drugs of the salicylic acid series such as various aspirin preparations including bafferin, those of the anthranilic acid series such as mefenamic acid and flufenamic acid, those of the phenylacetic acid series such as alclofanac, diclofenac and fenbufen, those of the indole series such as indomethacin, those of indene series such as sulindac, those of the heteroarylacetic acid series such as tolmecin, those of the propionic acid series such as ibuprofen, naproxen, ketoprofen and flurbiprofen, those of the pyrazolone series such as phenylbutazone, oxyphenbutazone, azapropazone, clofezone, ketophenylbutazone and succibutazone, those of the benzothiazine series such as piroxicam, those of the phenothiazine series such as metiazinic acid, those of the pyrimidine series such as bucolome, those of the indazole series such as benzydamine, those of the pyrimidinylpyrazole series such as mepirizol, those of the thienopyridine series such as tinoridine hydrochloride, and those of the benzothiazolinone series such as tiaramid hydrochloride. However, these drugs are weak in the main pharmacological activities and/or exhibit relatively high toxicity when compared with their main pharmacological activities so that a prolonged administration of the drugs will result in serious side-effects or drug-tolerance. In the case of the drugs of the salicylic acid series such as aspirin preparations, for example, the use in a smaller dose exhibits strong anti-inflammatory, antipyretic and analgesic activities while the use in a larger dose displays anti-rheumatic activity. On the other hand, prolonged use of these drugs causes tinnitus and seriously attacks the liver and digestive organs, especially the stomach. The use of drugs of the anthranilic acid series such as mefenamic acid exhibit analgesic activity but with poor anti-inflammatory activity and cause, on the other hand, lesion, diarrhoea and digestive system disorders. The use of drugs of the phenylacetic acid series produces relatively weak side-effects but exhibits only a medium degree of anti-inflammatory, antipyretic and analgesic activities. The use of drugs of the indole series such as indomethacin exhibits especially remarkable anti-rheumatic activity in addition to anti-inflammatory and analgesic activities. However these drugs influence the central nervous system resulting in headaches and giddiness, as well as side-effects in the digestive system. In case of drugs of the indene series such as sulindac, the side-effects in the central nervous system exhibited by drugs of the indole series are almost eliminated but a medium degree of digestive system disorder still remains as a side-effect. The use of drugs of the heteroarylacetic acid series exhibits potent analgesic activity but causes digestive system disorders. The use of drugs of the propionic acid series such as ibuprofen is now recommended because of their weak side-effects. However, these drugs do not have potent pharmacological effects although they do possess some anti-inflammatory, antipyretic and analgesic activities on average. Drugs of the pyrazolone series such as phenylbutazone exhibit various pharmacological effects in addition to anti-inflammatory and analgesic activities but cause various side-effects including lesion, edema, blood dyscrasia, hepatic disorder and digestive system disorders. Drugs of the benzothiazine series exhibit rapid and long lasting anti-inflammatory, antipyretic and analgesic activities of medium degree but also exhibit edema and digestive system disorders.

Thus, all of the drugs now commercially available which exhibit anti-inflammatory, antipyretic and analgesic activities are accompanied by several side-effects. If such side-effects are substantially eliminated, the main pharmacological effects are in the enhanced and the prolonged use of such drugs exhibiting potent pharmacological activities becomes possible. Thus, there is still great demand for developing a new compound which exhibits potent pharmacological activities with negligible side-effects.

In GB-A-0 070 013 the use of a (5-alkyl-2-oxo-1,3-dioxolen-4yl)methyl ester moiety to enhance the oral absorption of carboxylic acid pharmaceuticals is disclosed.

It is an object of the present invention to provide a new 2-anilinophenylacetic acid derivative possessing long lasting and potent valuable pharmacological activities with negligible side-effects.

It is another object of the present invention to provide a new non-steroidal anti-inflammatory and analgesic drug accompanied with a negligibly low level of toxicity and side-effects.

It is still another object of the present invention to provide a process for preparing such new compound.

With a view to develop a new type of anti-inflammatory drug which has potent and long lasting pharmacological effects with negligible side-effects, the present inventors have synthesized a series of new arylacetic acid derivatives and checked their pharmacological activities. As a result of such extensive

research, it has now been found that a new compound of the 2-anilinophenylacetic acid series having the formula:

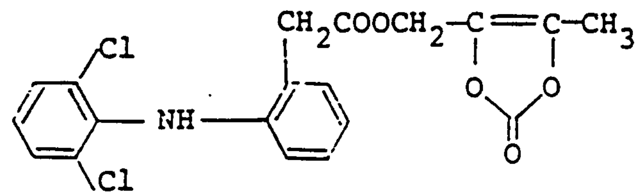

(I)

possesses extremely potent and remarkable anti-inflammatory and analgesic activities with negligible side-effects as represented by the lack of side effects in the digestive system. The present invention has been accomplished on the basis of the above finding.

In accordance with one and prime embodiment of this invention, there is provided (5-methyl-2-oxo-1,3-dioxolen-4-yl)methyl2-(2,6-dichloroanilino)phenylacetate of the formula:

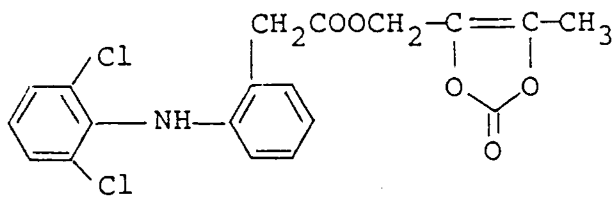

(I)

In accordance with another embodiment of this invention, there is provided a process for the preparation of (5-methyl-2-oxo-1,3-dioxolen-4-yl)methyl 2-(2,6-dichloroanilino)phenylacetate of the formula:

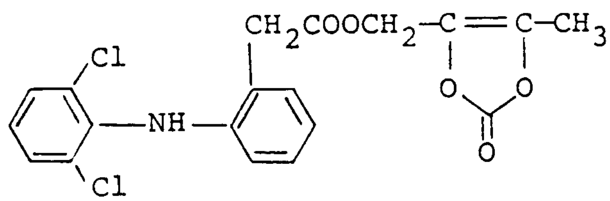

(I)

which comprises reacting a salt of 2-(2,6-dichloroanilino)phenylacetic acid of the formula:

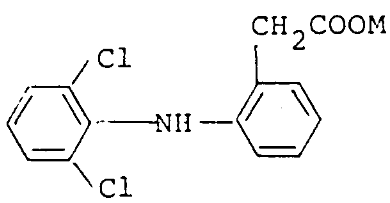

(II)

wherein M is an alkali metal or ammonium group of an organic tertiary amine,
with a 4-methyl-5-halogenomethyl-1,3-dioxolen-2-one of the formula:

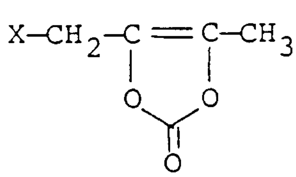

(III)

wherein X is a halogen atom.

The new compound of this invention is a white crystalline substance soluble in organic solvents such as alcohols and can be prepared by reacting the compound of the formula (II) with the compound of the formula (III) in a manner known per se for condensation.

The compound of the formula (II) and the compound of the formula (III) are both known compounds. The compound of the formula (II) is prepared, for example, according to the methods described in Japanese Patent Publn. No. Sho. 42-23418 by saponification of the corresponding ester, acid hydrolysis of the corresponding nitrile or alkaline hydrolysis of a lactam of the formula:

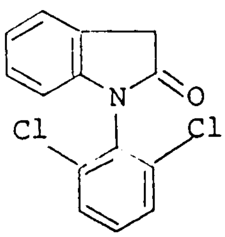

(IV).

The corresponding nitrile is prepared by reacting an α-halogeno-N-phenyl-o-toluidine such as α-chloro-N-(2,6-dichlorophenyl)-o-toluidine with an alkali metal cyanide such as KCN. The compound of the formula (IV) is prepared according to several methods, for example, by heating 2-chloro-N-(2,6-dichloro)-phenyl-acetanilide with aluminum chloride at about 160 °C or at 100-150 °C in tetrachloroethane or nitrobenzene.

The compound of the formula (III) is prepared, for example, according to a method described in JP-A-57-26684 by reacting 4,5-dimethyl-1,3-dioxolen-2-one with a chlorinating agent such as gaseous chlorine, N-chlorosuccinimide or N-chlorophthalimide under a radical-generating condition (irradiation of UV-rays or the use of a radical-generating agent such as a peroxide) at room temperature or an elevated temperature.

The new compound of this invention represented by the formula (I) can be prepared by condensing the compound of the formula (II) with the compound of the formula (III).

This condensation reaction may also be regarded as one mode of the esterification reaction wherein a functionally reactive carboxylic acid is reacted with a hydroxy compound, the hydroxyl group of which has been converted into a functionally reactive form. Thus, the reaction between the compound of the formula (II) and the compound of the formula (III) is carried out in the presence of an organic solvent optionally with a base as an acid-binding agent.

Any of the organic solvents can be used for the above reaction so far as the solvent does not influence the reaction. In general, ketones, ethers, nitriles and esters can conveniently be used as the solvent, but alkyl halides or similar reactive solvents are not recommended. Illustrative of the preferable solvents are, for example, acetone, methyl ethyl ketone, tetrahydrofuran, dioxane, dimethylformamide, diethylformamide, triethylamine and pyridine.

The use of the base as an acid-binding agent is not indispensable for the above reaction but is preferable as the reaction is promoted in the presence of a base. In the present invention, the utilizable base is not extremely strong. Illustrative of the bases used as acid-binding agents are, for example, sodium carbonate, potassium carbonate, sodium hydrogen carbonate, potassium hydrogen carbonate, triethylamine, pyridine and picoline. The use of a weak inorganic base such as sodium or potassium hydrogen carbonate is preferable. These bases are generally used in a smaller amount compared with the reactants, but triethylamine may be used in a larger amount as it may also serve as the reaction solvent.

No particular limitation exists in the proportion of the reactants. In general, however, the reactants are used in stoichiometric amount. The reaction solvent is used at least in an amount sufficient to form a solution or suspension of the reactants.

The reaction is carried out at a temperature ranging from -10 °C to the boiling point of the solvent used. The reaction temperature is preferably selected from the range from 10 °C to 80 °C. The reaction time varies according to the condition employed but is generally within the period of 1-3 hours.

The new 2-anilinophenylacetic ester thus obtained is separated from the reaction mixture and purified by recrystallization from an alcohol.

Results of various tests made on the pharmacological activities of the new compound of this invention, have shown that the new compound is comparable in anti-inflammatory activity with the known compounds which are now regarded as most potent in anti-inflammatory activity, but has extremely weak side-effects as represented by the lack of gastric ulcerogenic effects compared with the known compounds. Thus, the compound of this invention is in toto much more potent in anti-inflammatory and analgesic activities than

the known compounds. This fact is evident from the following tests on the pharmacological effects:

1. Tests of the pharmacological effects

(a) Inhibitory effect on carrageenin-induced hind paw edema in rats:

40 Male rats of Wistar strain weighing 100-130 g were divided into the following 5 groups each consisting of 8 rats: 1. Control group, 2. Group for the administration of the compound of this invention, 3. Group for the administration of diclofenac Na, 4. Group for the administration of flurubiprofen and 5. Group for the administration of indomethacin. Prior to the administration of the test compounds, plantar injections 1% carrageenin solution were made to each rat to induce hind paw edema. To each rat of the Groups 3, 4 and 5, the $LD_{50}$ amount of the relevant test compound was orally administered in molar amounts. The compound of the present invention was administered in an equimolar amount to the diclofenac sodium salt.

Comparison of inhibitory effects of the test compounds on hind paw edema induced by carrageenin showed that swelling of the edema was significantly inhibited in all of the Groups to which the test compounds were administered compared with the Control group ($P<0.01$). However, no significant difference in inhibitory effect was observed between Group 2 to which the compound of this invention was administered and all other Groups to which the other test compounds were administered. It has been shown therefore that the compound of this invention has comparable inhibitory effects on carrageenin-induced hind paw edema in rats with diclofenac sodium salt, indomethacin and flurubiprofen which are known to be potent anti-inflammatory drugs.

(b) Analgesic and anti-capillary permeability effects according to Wittle's method:

Three groups of female mice of ICR strain weighing 22-28 g were used, each group consisting of 10 mice; one group (Group 1) was used for the administration of the compound of this invention, another group (Group 2) for the administration of diclofenac sodium salt and the other group (Group 3) as control. Each test compound was orally administered at a dose of 25 mg/kg and 20 minutes after the oral administration, a 4% Pontamine Sky Blue (PSB) solution was intravenously injected to each mouse of Groups 1, 2 and 3, and 10 minutes after the injection, 1% acetic acid was intraperitoneally injected to each mouse. Immediately after the intraperitoneal injection, the number of stretchings of the treated mice were counted and recorded for 20 minutes. After this observation, the mice were immediately sacrificed by dislocation of cervical vertebrae and subjected to ventrotomy. The peritoneal cavity of each mouse was washed with 5 ml of distilled water to exudate PSB in the peritoneal cavity, and the amount of the colorant exudated was measured.

Results of the tests showed that Groups 1 and 2 displayed a significant difference in inhibitory activities of the stretching and exudation of the colorant from the peritoneal cavity compared with Group 3 ($P<0.01$). No significant difference in analgesic and anti-capillary permeability effects was found between Groups 1 and 2. Thus, the compound of this invention is comparable in these effects to diclofenac sodium salt.

2. Acute toxicity and gastric ulcerogenic actions

(a) Acute oral toxicity in mice:

Groups of male and female mice of ICR strain weighing 25-35 g were used for the test, each group consisting of 6 mice. The compound of this invention was orally administered in a dose of 250 mg/kg, 290 mg/kg, 336mg/kg, 390 mg/kg or 452 mg/kg to the mice of each group. $LD_{50}$ values of the compound of this invention were calculated for each concentration according to the Litchfield-Wilcoxon's method from the number of dead mice during one week. The $LD_{50}$ values for oral administration of the compound of this invention was determined to be 460 mg/kg for male mice and 516 mg/kg for female mice. These values were approximately 3 times as high as those of diclofenac sodium salt showing 145 mg/kg for male mice and 135 mg/kg for female mice.

(b) Gastric ulcerogenic action:

Three groups of male rats of Wistar strain weighing 300-340 g were used, each group consisting of 7 rats; one group (Group 1) was used for control, another group (Group 2) for the administration of the compound of this invention and the other group (Group 3) for the administration of diclofenac sodium salt.

After 24 hour fasting prior to the test, the compound of this invention and the diclofenac sodium salt were orally administered each in a dose of 20 mg/kg to the rats of the relevant groups. Six hours after the administration, the rats were sacrificed by decaptation and their stomachs were immediately removed. A 5% formaline solution (10 ml) was injected through the cardia into the stomach cavity and the stomach was fixed overnight in a 10% formaline solution. Each fixed stomach was cut along the greater curvature, and the inside of the stomach was investigated by the naked eye to check whether ulcers and erosions occurred or not. An ulcerogenic and erosion index was 0.71±0.42 in Group 1, 1.13±0.40 in Group 2 and 17.57±4.28 in Group 3. The number of rats having ulcers or erosions was 3/7 (42.9%) in Group 1, 4/7 (57.1%) in Group 2 and 7/7 (100%) in Group 3.

No significant difference was found between Group 1 and Group 2 with respect to the number of rats having ulcers or erosions. However, significant differences were found between Group 3 and Groups 1 and 2 in the ulcerogenic and erosion index and the number of rats having ulcers or erosions(P<0.01).

The above fact apparently shows that the gastric ulcerogenic side-effects of the compound of this invention are extremely weak and almost negligible compared with that of the diclofenac sodium salt which is one of the known potent anti-inflammatory drugs. It is evident therefore that the compound of this invention has more potent anti-inflammatory and analgesic activities compared with any other known potent similar drugs.

The present invention will now be illustrated in more detail by way of an example.

Example

6.5 Grams (20 m-mol) of sodium 2-(2,6-dichloroanilino)phenylacetate was dispersed in 45 ml of dimethylformamide, and 0.4 g (4 m-mol) potassium hydrogen carbonate was added to the dispersion. The dispersion was ice cooled and 4.3 g (22 m-mol) of 4-bromomethyl-5-methyl-1,3-dioxolen-2-one was then added dropwise. The mixture was stirred for one hour at room temperature and then for one hour at 60 °C. After completion of the reaction, the reaction liquid was cooled down to room temperature and then dispersed in ice water. The oily substance separated was extracted with benzene, and the extract was washed with water, dried and then concentrated by distilling off the solvents. The residual substance obtained was recrystallized from methanol whereby 6.9 g (yield: 85%) of (5-methyl-2-oxo-1,3-dioxolen-4-yl)-methyl 2-(2,6-dichloroanilino)phenylacetate was obtained as white crystals.

Melting point : 92 °C

| Analysis ($C_{19}H_{15}Cl_2NO_5$) | | | |
|---|---|---|---|
| Calcd. (%)<br>Found. (%) | C,56.16 ;<br>C,55.90 ; | H,3.71 ;<br>H,3.70 ; | N,3.47<br>N,3.43 |

IR (KBr, $\nu$cm$^{-1}$) : 3320 (NH)
1820 (ringed carbonyl ester)
1735, 1715 (ester)

NMR ($CDCl_3$,$\delta$ppm): 2.15 (3H,s,methyl)
3.85 (2H,s,-$CH_2$CO)

4.90 (2H,s, 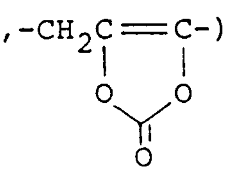 )

6.40-7.45 (8H,m,-NH and benzene ring)

**Claims**

**Claims for the following Contracting States : CH, DE, FR, GB, IT, LI, NL**

**1.** (5-Methyl-2-oxo-1,3-dioxolen-4-yl)methyl 2-(2,6-dichloroanilino)phenylacetate of the formula:

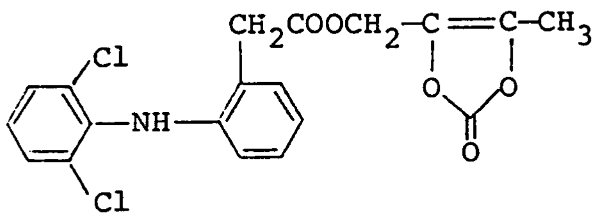

(I)

2. A process for the preparation of the compound according to claim 1 which comprises reacting a salt of 2-(2,6-dichloroanilino)phenylacetic acid of the formula:

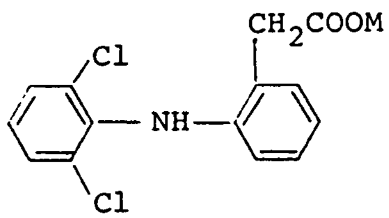

(II)

wherein M is an alkali metal or ammonium group of an organic tertiary amine, with a 4-methyl-5-halogenomethyl-1,3-dioxolen-2-one of the formula:

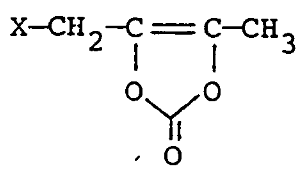

(III)

wherein X is a halogen atom.

3. The compound according to claim 1 for use as a pharmacological agent.

4. The compound according to claim 1 for use as a pharmacological agent having anti-inflammatory, antipyretic, analgetic and anti-rheumatic activity.

5. A pharmaceutical composition comprising the compound according to claim 1 and a physiologically acceptable carrier or diluent.

**Claims for the following Contracting State : ES**

1. A process for the preparation of (5-methyl-2-oxo-1,3-dioxolen-4-yl)methyl 2-(2,6-dichloroanilino)-phenylacetate of the formula:

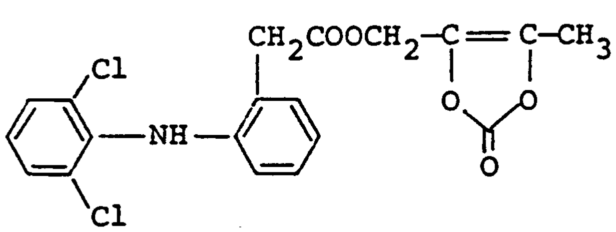

(I)

which comprises reacting a salt of 2-(2,6-dichloroanilino)phenylacetic acid of the formula:

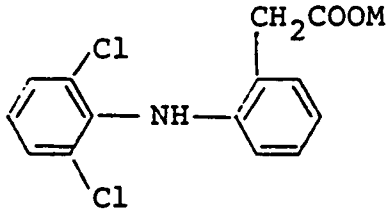

(II)

wherein M is an alkali metal or ammonium group of an organic tertiary amine, with a 4-methyl-5-halogenomethyl-1,3-dioxolen-2-one of the formula:

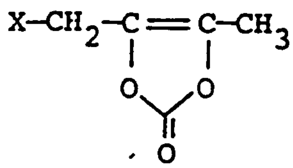

(III)

wherein X is a halogen atom.

2. A process for the preparation of a pharmaceutical composition wherein the compound prepared according to claim 1 is mixed with a pharmaceutically acceptable carrier or diluent.

3. The process according to claim 2 wherein the pharmaceutical composition has an anti-inflammatory, antipyretic, analgesia and anti-rheumatic activity.

**Patentansprüche**
**Patentansprüche für folgende Vertragsstaaten : CH, DE, FR, GB, IT, LI, NL**

1. (5-Methyl-2-oxo-1,3-dioxolen-4-yl)methyl-2-(2,6-dichloranilino)phenylacetat der Formel (I):

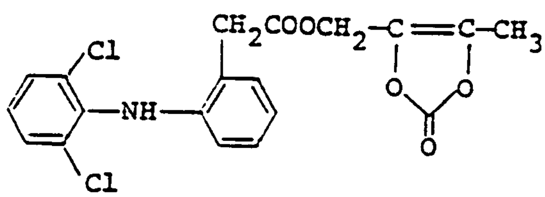

(I)

2. Verfahren zur Herstellung der Verbindung gemäß Anspruch 1, umfassend die Umsetzung eines 2-(2,6-Dichloranilino)phenylessigsäuresalzes der Formel (II):

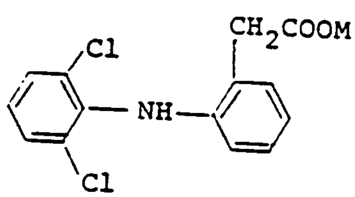

(II)

in der M ein Alkalimetall oder ein Ammoniumrest eines organischen tertiären Amins ist, mit einem 4-Methyl-5-halogenmethyl-1,3-dioxolen-2-on der Formel (III):

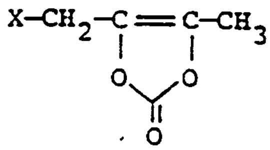

(III)

in der X ein Halogenatom ist.

3. Verbindung gemäß Anspruch 1 zur Verwendung als pharmakologischer Wirkstoff.

4. Verbindung gemäß Anspruch 1 zur Verwendung als pharmakologischer Wirkstoff mit entzündungshemmender, fiebersenkender, schmerzlindernder und antirheumatischer Aktivität.

5. Arzneimittel, umfassend die Verbindung gemäß Anspruch 1 und einen physiologisch verträglichen Träger oder ein Verdünnungsmittel.

**Patentansprüche für folgenden Vertragsstaat : ES**

1. Verfahren zur Herstellung von (5-Methyl-2-oxo-1,3-dioxolen-4-yl)methyl-2-(2,6-dichloranilino)-phenylacetat der Formel (I):

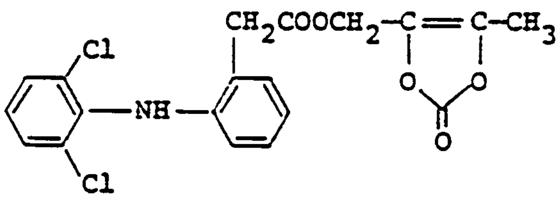

(I)

umfassend die Umsetzung eines 2-(2,6-Dichloranilino)phenylessigsäuresalzes der Formel (II):

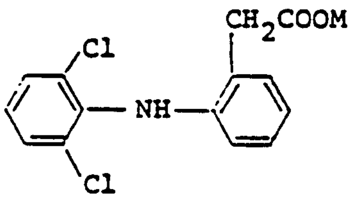

(II)

in der M ein Alkalimetall oder ein Ammoniumrest eines organischen tertiären Amins ist, mit einem 4-Methyl-5-halogenmethyl-1,3-dioxolen-2-on der Formel (III):

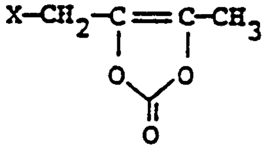

(III)

in der X ein Halogenatom ist.

2. Verfahren zur Herstellung eines Arzneimittels, wobei die nach Anspruch 1 hergestellte Verbindung mit einem pharmazeutisch verträglichen Träger oder einem Verdünnungsmittel gemischt ist.

3. Verfahren gemäß Anspruch 2, wobei das Arzneimittel eine entzündungshemmende, fiebersenkende, schmerzlindernde und antirheumatische Aktivität aufweist.

## Revendications

**Revendications pour les Etats contractants suivants : CH, DE, FR, GB, IT, LI, NL**

1. 2-(2,6-dichloroanilino)phénylacétate de (5-méthyl-2-oxo-1,3-dioxolène-4-yl)méthyle de formule :

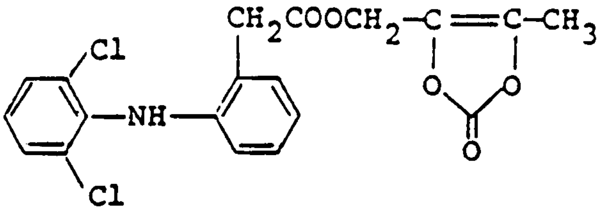

(I)

2. Un procédé pour la préparation du composé selon la revendication 1, qui comprend la réaction d'un sel de l'acide 2-(2,6-dichloroanilino)phénylacétique de formule :

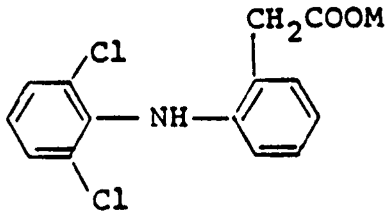

(II)

dans laquelle M est un métal alcalin ou un groupe ammonium d'une amine organique tertiaire, avec une 4-méthyl-5-halogénométhyl-1,3-dioxolène-2-one de formule :

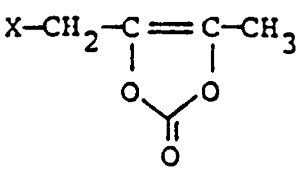

(III)

dans laquelle X est un atome d'halogène.

3. Le composé selon la revendication 1 pour l'emploi comme agent pharmacologique.

4. Le composé selon la revendication 1 pour l'emploi comme agent pharmacologique ayant une activité anti-inflammatoire, antipyrétique, analgésique et antirhumatismale.

5. Une composition pharmaceutique comprenant le composé selon la revendication 1 et un véhicule ou diluant physiologiquement acceptables.

**Revendications pour l'Etats contractant suivant : ES**

1. Un procédé pour la préparation du 2-(2,6-dichloroanilino) phénylacétate de (5-méthyl-2-oxo-1,3-dioxolène-4-yl)méthyle de formule :

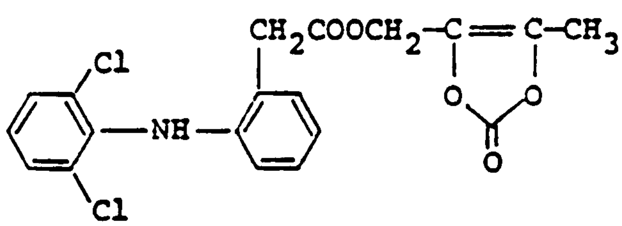

(I)

qui comprend la réaction d'un sel de l'acide 2-(2,6-dichloroanilino)phénylacétique de formule :

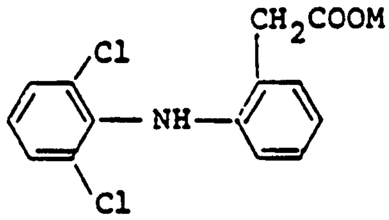

(II)

dans laquelle M est un métal alcalin ou un groupe ammonium d'une amine organique tertiaire, avec une 4-méthyl-5-halogénométhyl-1,3-dioxolène-2-one de formule :

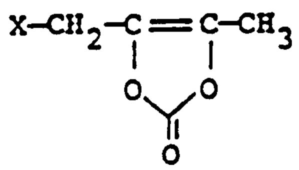

(III)

dans laquelle X est un atome d'halogène.

**2.** Un procédé pour la préparation d'une composition pharmaceutique, dans lequel on mélange le composé préparé selon la revendication 1 avec un véhicule ou diluant pharmaceutiquement acceptables.

**3.** Le procédé selon la revendication 2, dans lequel la composition pharmaceutique a une activité anti-inflammatoire, antipyrétique, analgésique et antirhumatismale.